(19) Europäisches Patentamt
European Patent Office
Office européen des brevets

(11) Numéro de publication: **0 068 999**
**B1**

(12) # FASCICULE DE BREVET EUROPEEN

(45) Date de publication du fascicule du brevet: **22.05.85**

(21) Numéro de dépôt: **82401129.0**

(22) Date de dépôt: **21.06.82**

(51) Int. Cl.⁴: **C 07 C 103/28**, C 07 C 103/29, C 07 C 103/76, C 07 D 295/14, C 07 D 295/18, A 61 K 31/165, A 61 K 31/395

(54) **Dérivés d'aryl-1 aminométhyl-2 cyclopropanes carboxamides (Z), leur préparation et leur application en tant que médicaments utiles dans le traitement des troubles du système nerveux central.**

(30) Priorité: **23.06.81 FR 8112312**

(43) Date de publication de la demande: **05.01.83 Bulletin 83/1**

(45) Mention de la délivrance du brevet: **22.05.85 Bulletin 85/21**

(84) Etats contractants désignés: **AT BE CH DE GB IT LI LU NL SE**

(56) Documents cités:
**FR - A - 2 208 661**

**CHEMICAL ABSTRACTS, vol.90, no.11, 12 mars 1979, page 561, résumé no.86788f, Columbus, Ohio (US) S. CASADIO et al.: "1-Phenyl-2-(hydroxymethyl) cyclopropanecarboxylic acid and derivatives"**

(73) Titulaire: **PIERRE FABRE S.A., 125, rue de la Faisanderie, F-75116 Paris (FR)**

(72) Inventeur: **Mouzin, Gilbert, 21 rue Sainte-Foy, F-81100 Castres (FR)**
Inventeur: **Cousse, Henri, La Foun de los Nobios Chemin de Lastinos, F-81100 Castres (FR)**
Inventeur: **Bonnaud, Bernard, 2, rue Georges Bizet, F-81100 Castres (FR)**
Inventeur: **Morre, Michel, Les Buis, Appartement 57, 11 rue Ste Odile, F-31100 Toulouse (FR)**
Inventeur: **Stenger, Antoine, 11 rue des Cigales, F-81100 Castres (FR)**

(74) Mandataire: **Corre, Jacques Denis Paul et al, Cabinet Regimbeau 26, Avenue Kléber, F-75116 Paris (FR)**

**EP 0 068 999 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention, réalisée au Centre de Recherches Pierre FABRE, concerne de nouveaux dérivés d'aryl-1 aminométhyl-2 cyclopropanes carboxamides (Z), leur procédé de préparation et leur utilisation en thérapeutique et en particulier dans le traitement des troubles du système nerveux central.

La technique antérieure la plus proche connue peut par exemple être illustrée par le brevet français n° 75 07120 de la demanderesse concernant un procédé de préparation d'acides aryl-1 hydroxyméthyl-2 cyclopropanes carboxyliques.

Ces dérivés acide-alcools ont par ailleurs fait l'objet d'une publication de G. Mouzin, H. Cousse, B. Bonnaud dans Synthesis 1978, 304, reprise dans Synthetic Methods or Organic Chemistry (Ed. W. Theilheimer) 34, 1980, 317.

L'étude pharmacologique des dérivés de l'acide phényl-1 hydroxyméthyl-2 cyclopropane carboxylique, décrits par S. Casadio, B. Bonnaud, G. Mouzin et H. Cousse dans Boll. Chim. Farm. 117, 1978, 331, a montré la faible activité pharmacologique de ces dérivés.

L'état de la technique antérieure peut encore être complété par la citation de FR-A-2 208 661 qui concerne toutefois des dérivés cyclopropaniques de structure et de profil pharmacologique différents.

La présente invention se rapporte à des composés qui se distinguent de ceux de la technique antérieure précitée; il s'agit d'amino amides cyclopropaniques répondant à la formule générale:

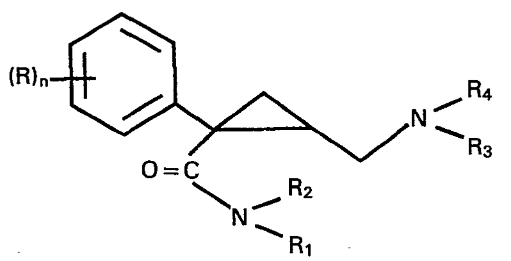

Il a en effet été démontré que certaines modulations effectuées au niveau des groupes fonctionnels portés par le groupe cyclopropanique conféraient à ces nouveaux amino amides cyclopropaniques de forts intéressantes propriétés pharmacologiques et plus particulièrement une action antidépressive très nette, qui permet d'utiliser ces composés en thérapeutique dans le traitement des troubles du système nerveux central.

La présente invention concerne de nouveaux dérivés d'aryl-1 aminométhyl-2 cyclopropanes carboxamides (Z) de formule générale I:

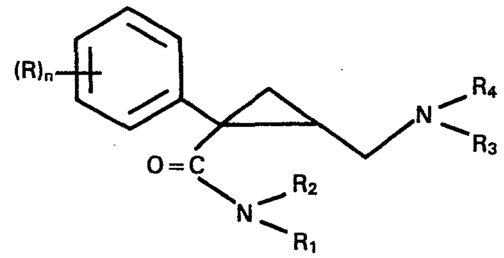

(I)

dans laquelle:

R représente un atome d'hydrogène ou d'halogène, un groupe alcoyle inférieur, alcoxy inférieur, hydroxy, nitro et amino;

n représente les valeurs 1 ou 2;

$R_1$ et $R_2$ représentent un atome d'hydrogène, un groupe alcoyle inférieur, ou un groupe aryle ou alcoylaryle inférieur, éventuellement substitués, de préférence en position para, par un atome d'halogène, en préférence par un atome de chlore;

$R_1$ et $R_2$ pouvant également former avec l'atome d'azote voisin un hétérocycle à 5 ou 6 chaînons;

$R_3$ et $R_4$ représentent un atome d'hydrogène ou un groupe alcoyle inférieur;

$R_3$ et $R_4$ pouvant également former avec l'atome d'azote voisin un hétérocycle à 5 ou 6 chaînons, contenant évetuellement un hétéroatome supplémentaire choisi parmi l'azote et l'oxygène.

La présente invention concerne également les sels des composés de formule générale I avec des acides minéraux ou organiques thérapeutiquement acceptables.

A titre d'exemples non limitatifs de ces sels on mentionnera les halogénohydrates, tels que chlorhydrate, fumarate, maléate, oxalate, citrate et le glutamate.

Dans la présente description, les radicaux alcoyle inférieur et les fragments alcoyle inférieur des radicaux alcoylaryle et alcoxy désignent des chaînes hydrocarbonés linéaires ou ramifiées, contenant de 1 à 4 atomes de carbone.

La présente invention concerne également un procédé de préparation des composés de formule générale I, consistant à hydrolyser un amino ester de formule générale (II), pour obtenir un amino-acide de formule générale (III).

Ledit amino acide de formule générale (III) est à son tour transformé en halogénure correspondant de formule générale (IV) au moyen d'un halogénure de thionyle. L'halogénure de formule générale (IV) est ensuite condensé sur une amine.

Le procédé de préparation conforme à la présente invention peut être illustré par le schéma réactionnel ci-après:

SCHEMA REACTIONNEL

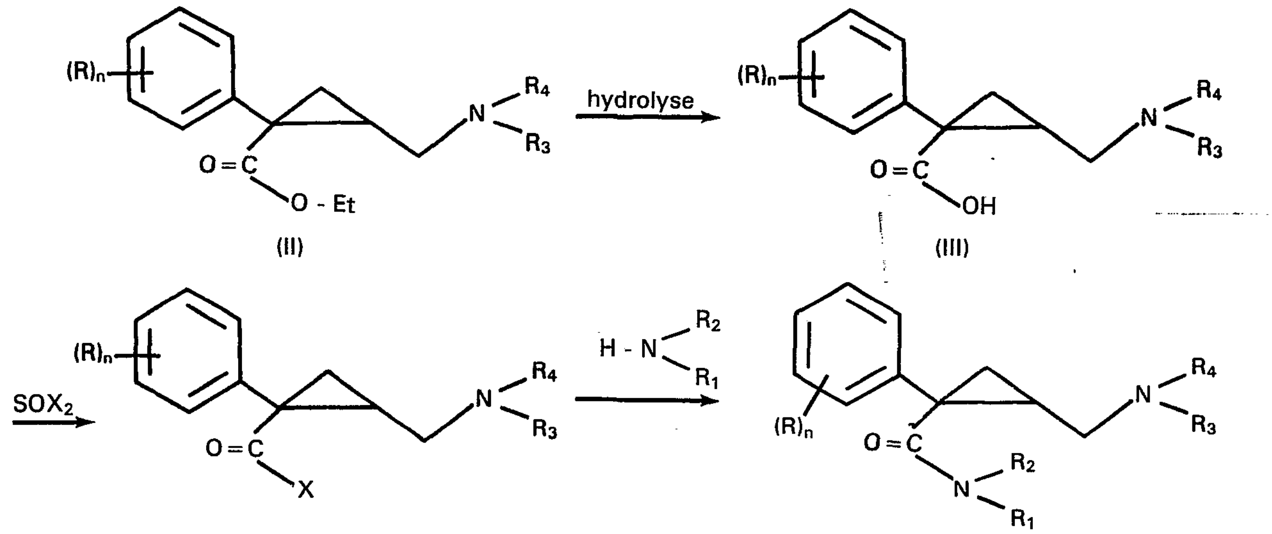


(II) (III) (IV) (I)

X représente un atome d'halogène, et

R, $R_1$, $R_2$, $R_3$, $R_4$ et n ont les significations données précédemment à propos de la formule générale I.

Les exemples mentionnés ci-après illustrent l'invention sans bien entendu en limiter la portée.

*Exemple 1*

Préparation du phényl-1 aminocarbonyl-1 diméthylamino méthyl-2 cyclopropane (Z)

a) *chlorhydrate de phényl-1 carboxy-1 diméthylamino méthyl-2 cyclopropane (Z)*

A une solution de 21,15 g de chlorhydrate de phényl-1 éthoxy carbonyl-1 diméthylaminométhyl-2 cyclopropane (Z) dans 200 $cm^3$ d'éthanol, on ajoute 180 $cm^3$ de soude (N). La solution est chauffée au reflux pendant 4 heures puis est neutralisée par addition de 105 $cm^3$ d'acide chlorhydrique (N). On évapore jusqu'à siccité et l'on reprend par de l'éthanol, on chlorhydrate et on précipite par addition d'éther éthylique.

On récupère avec un rendement de 85% le produit de formule:

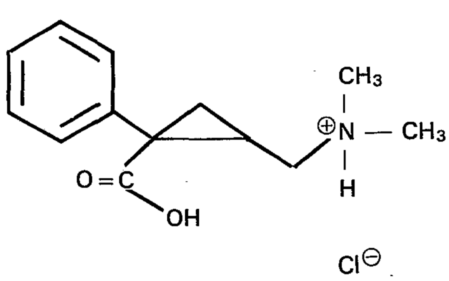

Formule brute: $C_{13}H_{18}Cl\,N\,O_2$
Masse moléculaire: 255,7
Cristaux: blancs
Point de fusion: 200°C

Chromatographie sur plaque:
— support: gel de silice 60 F 254 Merck
— solvant: butanol-acide acétique-eau 6/2/2
— révélation: UV et iode
— Rf: 0,33.

b) *chlorhydrate de phényl-1 chloro carbonyl-1 diméthylamino méthyl-2 cyclopropane (Z)*

On maintient sous agitation à température ambiante pendant 12 h, un mélange de 17,5 g de chlorhydrate de phényl-1 carboxy-1 diméthylaminométhyl cyclopropane (Z) dans 26 $cm^3$ de chlorure de thionyle. Puis on ajoute 150 $cm^3$ d'éther éthylique, le produit cristallise et on récupère avec un rendement de 95% le produit de formule:

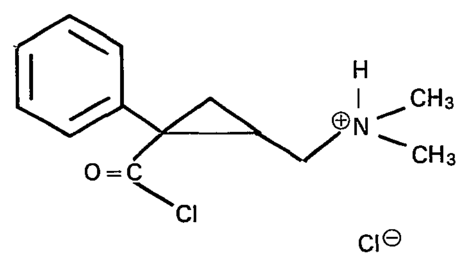

Formule brute: $C_{13}H_{17}Cl_2\,N\,O$
Masse moléculaire: 274,2
Cristaux: blancs
Point de fusion: 135°C.

c) *phényl-1 amino carbonyl-1 diméthylaminométhyl-2 cyclopropane (Z)*

Dans 15 $cm^3$ d'ammoniaque à 20% on introduit sous agitation et à température ambiante 5,48 g de chlorhydrate de phényl-1 diméthyl aminométhyl-2 cyclopropane (Z) puis on maintient 4 heures à température ambiante. Après les traitements habituels, on

récupère par recristallisation dans l'éther de pétrole avec un rendement de 75% le produit de formule:

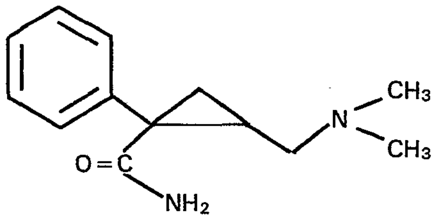

Formule brute: $C_{13}H_{18}N_2O$
Masse moléculaire: 218,29
Cristaux: blancs
Point de fusion: 84°C
Chromatographie sur plaque:
— support: gel de silice 60 F 254 Merck
— solvant: chloroforme-méthanol-ammoniaque 80/18/2
— révélation: UV et iode
— Rf: 0,44
Spectre IR (KBr): $\nu$ C=O 1670 $cm^{-1}$.

*Exemple 2*

*Préparation du phényl-1 diméthylamino carbonyl-1 diméthylamino méthyl-2 cyclopropane (Z)*

D'une façon similaire à celle décrite dans l'exemple 1, mais en utilisant la diméthylamine et en salifiant par l'acide chlorhydrique on obtient le produit de formule:

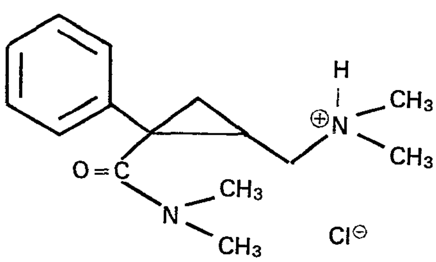

Formule brute: $C_{15}H_{23}ClN_2O$
Masse moléculaire: 282,8
Cristaux: blancs
Point de fusion: 210°C
Chromatographie sur plaque:
— support: gel de silice 60 F 254 Merck
— solvant: chloroforme-méthanol-ammoniaque 80/18/2
— révélation: UV et iode
— Rf: 0,52
Spectre IR (KBr): $\nu$ C=O 1630 $cm^{-1}$.

*Exemple 3*

*Maléate de phényl-1 éthylamino carbonyl-1 diméthylamino méthyl-2 cyclopropane (Z)*

D'une façon similaire à celle décrite dans l'exemple 1, mais en utilisant l'éthylamine et l'acide maléique comme agent salifiant on obtient le produit de formule:

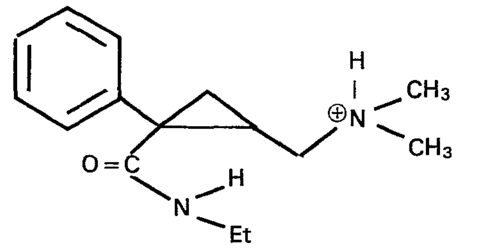

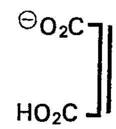

Formule brute: $C_{19}H_{26}N_2O_5$
Masse moléculaire: 362,4
Cristaux: blancs
Point de fusion: 105°C
Chromatographie sur plaque:
— support: gel de silice 60 F 254 Merck
— solvant: chloroforme-méthanol-ammoniaque 80/18/2
— révélation: UV et iode
— Rf: 0,58
Spectre IR (KBr): $\nu$ C=O (amide) 1645 $cm^{-1}$.

*Exemple 4*

*Chlorhydrate de phényl-1 diéthylaminocarbonyl-1 amino méthyl-2 cyclopropane (Z)*

D'une façon similaire à celle décrite dans l'exemple 1, mais en hydrolysant le chlorhydrate de phényl-1 éthoxy carbonyl-1 aminométhyl-2 cyclopropane (Z) puis en traitant le chlorure d'acide intermédiaire par la diéthylamine et en salifiant par l'acide chlorhydrique, on obtient le produit de formule:

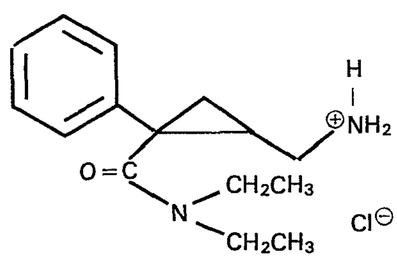

Formule brute: $C_{15}H_{23}ClN_2O$
Masse moléculaire: 282,8
Cristaux: blancs
Point de fusion: 180°C
Chromatographie sur plaque:
— support: gel de silice 60 F 254 Merck
— solvant: butanol-acide acétique-eau 6/2/2
— révélation: UV et iode
— Rf: 0,44
Spectre IR (KBr): $\nu$ C=O 1620 $cm^{-1}$.

*Exemple 5*

*Préparation du phényl-1 diméthylaminométhyl-2 N(4' chlorophényl) cyclopropane carboxamide (Z) chlorhydrate*

D'une façon similaire à celle décrite dans l'exemple 1, mais en utilisant la parachloroaniline et en salifiant par l'acide chlorhydrique on obtient le produit de formule:

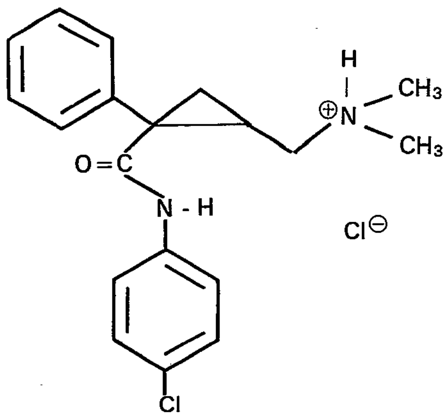

Formule brute: $C_{19}H_{22}Cl_2N_2O$
Masse moléculaire: 365,3
Cristaux: blancs
Point de fusion: 226°C
Chromatographie sur plaque:
— support: gel de silice 60 F 254 Merck
— solvant: butanol-acide acétique-eau 6/2/2
— révélation: UV et iode
— Rf: 0,46
Spectre IR (KBr): $\nu$ C=O 1665 $cm^{-1}$.

*Exemple 6*

*Préparation du phényl-1 diméthylaminométhyl-2 N(4' chloro benzyl) cyclopropane carboxamide (Z) maléate*

D'une façon similaire à celle décrite dans l'exemple 1, mais en utilisant la parachlorobenzylamine et en salifiant avec l'acide maléique, on obtient le produit de formule:

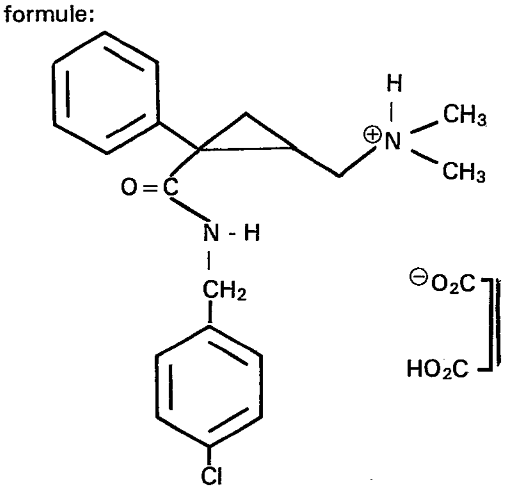

Formule brute: $C_{24}H_{27}Cl\ N_2\ O_5$
Masse moléculaire: 458,9
Cristaux: blancs
Point de fusion: 110°C
Chromatographie sur plaque:
— support: gel de silice 60 F 254 Merck
— solvant: chloroforme-méthanol-ammoniaque 80/18/2
— révélation: UV et iode
— Rf: 0,76
Spectre IR (KBr): $\nu$ C=O 1640 $cm^{-1}$ (amide).

*Exemple 7*

*Préparation du chlorhydrate de phényl-1 diméthylamino méthyl-2 N(phényl-2 éthyl) cyclopropane carboxamide (Z)*

D'une façon similaire à celle décrite dans l'exemple 1, mais en utilisant la phényl-2 éthylamine et en salifiant avec l'acide chlorhydrique, on obtient le produit de formule:

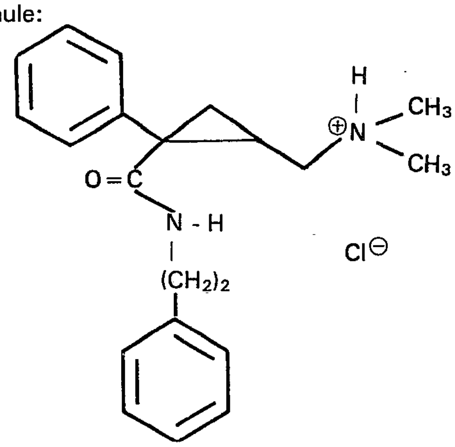

Formule brute: $C_{21}H_{27}Cl\ N_2\ O$
Masse moléculaire: 358,9
Cristaux: blancs
Point de fusion: 160°C
Chromatographie sur plaque:
— support: gel de silice 60 F 254 Merck
— solvant: butanol-acide acétique-eau 6/2/2
— révélation: UV et iode
— Rf: 0,45
Spectre IR (KBr): $\nu$ C=O 1650 $cm^{-1}$.

*Exemple 8*

*Préparation du chlorhydrate de (dichloro-3-4 phényl-1) diméthylaminométhyl-2, N-N-diméthyl cyclopropane carboxamide (Z)*

D'une façon similaire à celle décrite dans l'exemple 1, mais en utilisant le chlorhydrate de (dichloro-3-4 phényl)-1 éthoxy carbonyl-1 diméthylamino méthyl-2 cyclopropane (Z) puis en traitant le chlorure d'acide intermédiaire par la diméthylamine, on obtient le produit de formule:

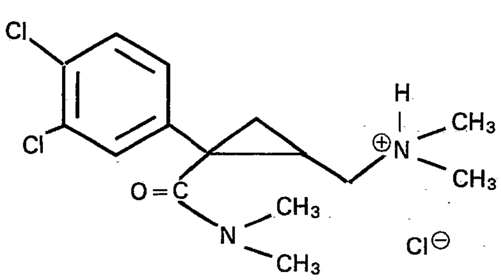

Formule brute: $C_{15}H_{21}Cl_3N_2O$
Masse moléculaire: 351,7
Cristaux: blancs
Point de fusion: 239 - 240°C
Chromatographie sur plaque:
- support: gel de silice 60 F 254 Merck
- solvant: butanol-acide acétique-eau 6/2/2
- révélation: UV et iode
- Rf: 0,27

Spectre IR (KBr): $\nu$ C=O 1630 $cm^{-1}$.

*Exemple 9*

*Préparation du chlorhydrate de phényl-1 pyrrolidino carbonyl-1 morpholino méthyl-2 cyclopropane (Z)*

D'une façon similaire à celle décrite dans l'exemple 1, mais en hydrolysant le chlorhydrate de phényl-1 éthoxy carbonyl-1 morpholino méthyl-2 cyclopropane (Z) puis en traitant le chlorure d'acide intermédiaire par la pyrrolidine, on obtient le produit de formule:

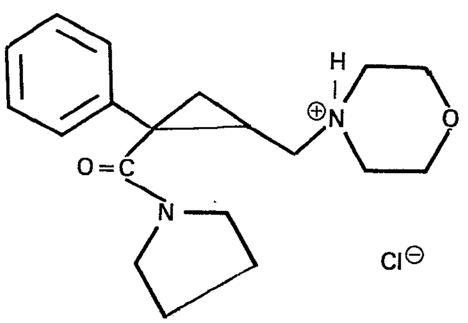

Formule brute: $C_{19}H_{27}Cl\,N_2O_2$
Masse moléculaire: 350,9
Cristaux: jaune pâle
Point de fusion: 260°C
Chromatographie sur plaque:
- support: gel de silice 60 F 254 Merck
- solvant: butanol-acide acétique-eau 6/2/2
- révélation: UV et iode
- Rf: 0,25

Spectre IR (KBr): $\nu$ C=O 1630 $cm^{-1}$.

*Exemple 10*

*Préparation du chlorhydrate de p-chlorophényl-1 aminocarbonyl-1 aminométhyl-2 cyclopropane (Z)*

D'une façon similaire à celle décrite dans l'exemple 1, mais en hydrolysant le chlorhydrate de p-chlorophényl-1 éthoxy carbonyl-1 aminométhyl-2 cyclopropane (Z), puis en traitant le chlorure d'acide intermédiaire par l'ammoniaque et en salifiant par l'acide chlorhydrique, on obtient le produit de formule:

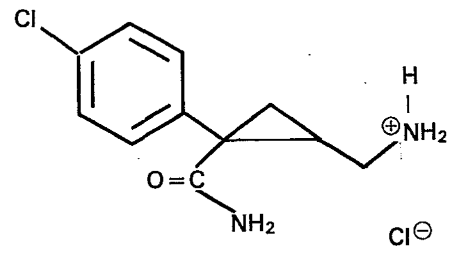

Formule brute: $C_{11}H_{14}Cl_2N_2O$
Masse moléculaire: 261,15.

*Exemple 11*

*Préparation du chlorhydrate d'orthochlorophényl-1 amino carbonyl-1 diméthylamino méthyl-2 cyclopropane (Z)*

D'une façon similaire à celle décrite dans l'exemple 1, mais en hydrolysant le chlorhydrate d'orthochlorophényl-1 éthoxy carbonyl-1 diméthylaminométhyl-2 cyclopropane (Z), puis en traitant le chlorure d'acide intermédiaire par l'ammoniaque et en salifiant par l'acide chlorhydrique, on obtient le produit de formule:

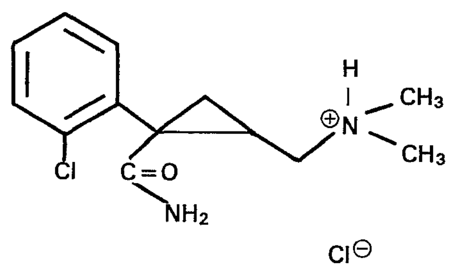

Formule brute: $C_{13}H_{18}Cl_2N_2O$
Masse moléculaire: 289,21.

*Exemple 12*

*Préparation du chlorhydrate de p-hydroxy phényl-1 amino carbonyl-1 diméthylaminométhyl-2 cyclopropane (Z)*

D'une façon similaire à celle décrite dans l'exemple 1, mais en hydrolysant le p-hydroxyphényl-1 éthoxy carbonyl-1 diméthylaminométhyl-2 cyclopropane (Z), puis en traitant le chlorure d'acide intermédiaire par l'ammoniaque et en salifiant par l'acide chlorhydrique, on obtient le produit de formule:

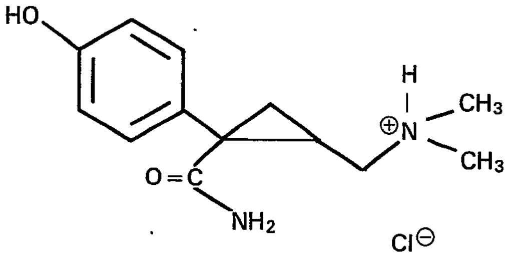

Formule brute: $C_{13}H_{19}Cl\ N_2O_2$
Masse moléculaire: 270,76.

*Exemple 13*

*Préparation du chlorhydrate de p-nitro phényl-1 diméthyl aminocarbonyl-1 diméthylaminométhyl--2 cyclopropane (Z)*

D'une façon similaire à celle décrite dans l'exemple 1, mais en hydrolysant le p-nitrophényl-1 éthoxy carbonyl-1 diméthylamino méthyl-2 cyclopropane (Z) puis en traitant le chlorure d'acide intermédiaire par la diméthylamine et en salifiant par l'acide chlorhydrique, on obtient le produit de formule:

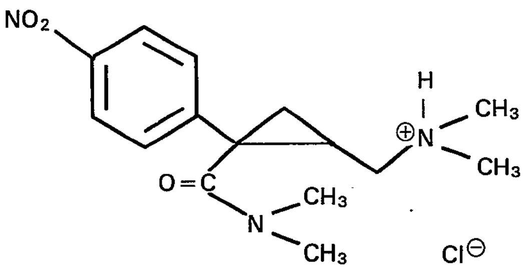

Formule brute: $C_{15}H_{22}Cl\ N_3O_3$
Masse moléculaire: 327,8.

*Exemple 14*

*Préparation du chlorhydrate de p-aminophényl-1 diméthyl aminocarbonyl-1 diméthylaminométhyl--2 cyclopropane (Z)*

D'une façon similaire à celle décrite dans l'exemple 1, mais en hydrolysant le p-aminophényl-1 éthoxy-carbonyl-1 diméthyl aminométhyl-2 cyclopropane (Z) puis en traitant le chlorure d'acide intermédiaire par la diméthylamine et en salifiant par l'acide chlorhydrique, on obtient le produit de formule:

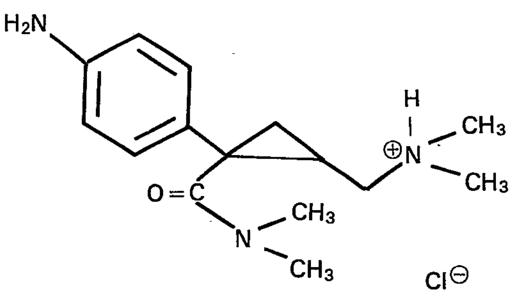

Formule brute: $C_{15}H_{24}Cl\ N_3O$
Masse moléculaire: 297,8.

*Exemple 15*

*Préparation du chlorhydrate de p-tolyl-1 méthyl-amino carbonyl-1 diméthylamino méthyl-2 cyclopropane (Z)*

D'une façon similaire à celle décrite dans l'exemple 1, mais en hydrolysant le p-tolyl-1 éthoxy carbonyl-1 diméthylaminométhyl-2 cyclopropane (Z) puis en traitant le chlorure d'acide intermédiaire par la diméthylamine et en salifiant par l'acide chlorhydrique, on obtient le produit de formule:

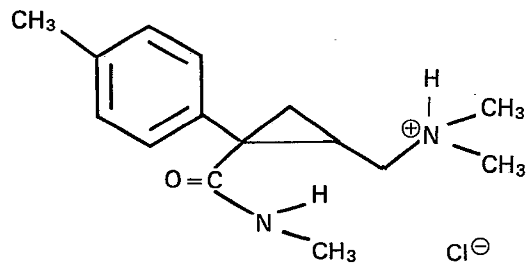

Formule brute: $C_{15}H_{23}Cl\ N_2\ O$
Masse moléculaire: 282,8.

*Exemple 16*

*Préparation du chlorhydrate de p-méthoxy phényl-1 amino carbonyl-1 aminométhyl-2 cyclopropane (Z)*

D'une façon similaire à celle décrite dans l'exemple 1, mais en hydrolysant le p-méthoxy phényl-1 éthoxy carbonyl-1 amino méthyl-2 cyclopropane (Z) puis en traitant le chlorure d'acide intermédiaire par l'ammoniaque et en salifiant par l'acide chlorhydrique, on obtient le produit de formule:

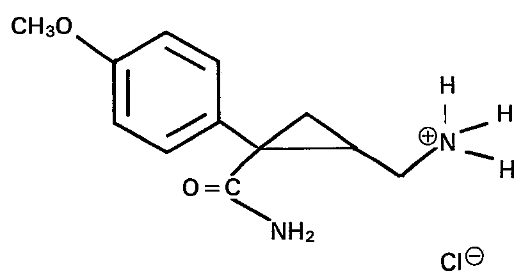

Formule brute: $C_{12}H_{17}Cl\ N_2\ O_2$
Masse moléculaire: 256,7.

*Expérimentations*

a) *Toxicologie*

Les composés chimiques précédemment décrits ont été soumis à des contrôles de toxicité.

Cette étude a été effectuée chez la souris conventionnelle pesant de 20 à 22 grammes.

Les substances ont été administrées par voie orale.

La $DL_{50}$ est calculée selon la méthode de KARBER, Arch. Exptl. Pathol. Pharmacol. 1931, 162, 480.

Toutes les $DL_{50}$ observées sont comprises entre 300 et 1000 mg/kg.

b) *Etude pharmacologique*

Les expérimentations pharmacologiques auxquelles ont été soumises les molécules chimiques, objet de la présente invention, ont permis de mettre en évidence une activité antidépressive.

A titre d'exemple particulier on citera le composé de l'exemple 4, le chlorhydrate de phényl-1 diéthylaminocarbonyl-1 aminométhyl-2 cyclopropane (Z), qui a une $DE_{50}$ de 0,1 mg/kg sur le test de potentialisation de la toxicité de la yohimbine, alors que la désimipramine prise comme référence a une $DE_{50}$ de 1,6 mg/kg.

c) *Application thérapeutique*

Compte tenu de leurs propriétés pharmacologiques et de leur faible toxicité, ces composés peuvent être utilisés en thérapeutique dans le traitement des troubles du système nerveux central; le composé de l'exemple 4, le chlorhydrate de phényl-1 diéthylaminocarbonyl-1 aminométhyl-2 cyclopropane (Z), ayant conduit à des résultats tout particulièrement intéressants.

Les composés de formule générale I selon la présente invention et leurs sels d'addition avec des acides thérapeutiquement compatibles peuvent ainsi être utilisés comme médicaments, par exemple sous forme de préparations pharmaceutiques adaptées facilitant la biodisponibilité. Ces préparations peuvent se présenter sous forme solide par exemple de comprimés, dragées, capsules, gélules ou sous forme liquide, par exemple de solutions, suspensions ou émulsions. Les préparations pharmaceutiques sous une forme appropriée à l'injection sont soumises à des opérations pharmaceutiques classiques, telles que stérilisation et/ou peuvent contenir des adjuvants par exemple des agents conservateurs, stabilisants, de mouillage ou d'émulsification, des comprimés tampons, etc.

Les dosages auxquels les composés actifs et leurs sels d'addition peuvent être administrés, peuvent varier dans des proportions importantes selon l'état du patient.

Un dosage quotidien d'environ 0,1 mg à 1 mg/kg de poids corporel est toutefois préféré.

Les compositions pharmaceutiques selon l'invention peuvent être utilisées en médecine humaine et vétérinaire et plus particulièrement dans le traitement d'états dépressifs névrotiques et réactionnels de diverses natures.

Bien entendu, la présente invention ne se trouve pas limitée aux exemples particuliers mentionnés à simple titre illustratif, mais il est parfaitement possible sans pour autant sortir du cadre de l'invention d'en imaginer un certain nombre de variantes et de modifications.

**Revendications pour les Etats contractants:**
BE, CH, DE, GB, IT, LI, LU, NL, SE

1. Dérivés d'aryl-1 aminométhyl-2 cyclopropanes carboxamides (Z) répondant à la formule générale I:

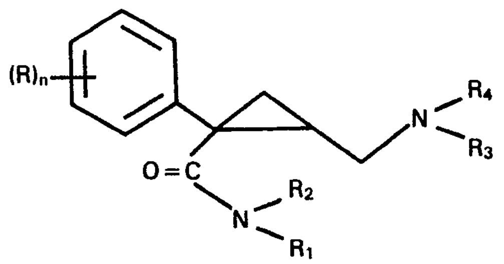

(I)

dans laquelle:

R représente un atome d'hydrogène ou d'halogène, un groupe alcoyle inférieur en $C_1$ à $C_4$, alcoxy inférieur en $C_1$ à $C_4$, hydroxy, nitro et amino;

n représente les valeurs 1 ou 2;

$R_1$ et $R_2$ représentent un atome d'hydrogène, un groupe alcoyle inférieur en $C_1$ à $C_4$, un groupe aryle ou alcoylaryle dont le fragment alcoyle contient 1 à 4 atomes de carbone, éventuellement substitués, de préférence en position para, par un atome d'halogène, de préférence par un atome de chlore;

$R_1$ et $R_2$ pouvant également former avec l'atome d'azote voisin un hétérocycle à 5 ou 6 chaînons;

$R_3$ et $R_4$ représentent un atome d'hydrogène ou un groupe alcoyle inférieur en $C_1$ à $C_4$;

$R_3$ et $R_4$ pouvant également former avec l'atome d'azote voisin un hétérocycle à 5 ou 6 chaînons, contenant éventuellement un hétéroatome supplémentaire choisi parmi l'azote et l'oxygène,

ainsi que leurs sels avec des acides minéraux ou organiques thérapeutiquement acceptables.

2. Le chlorhydrate de phényl-1 diéthylaminocarbonyl-1 aminométhyl-2 cyclopropane (Z) selon la revendication 1.

3. Le chlorhydrate de p-chlorophényl-1 aminocarbonyl-1 aminométhyl-2 cyclopropane (Z) selon la revendication 1.

4. Le chlorhydrate de p-méthoxy phényl-1 amino carbonyl-1 aminométhyl-2 cyclopropane (Z) selon la revendication 1.

5. Procédé de préparation des composés de formule générale I selon l'une des revendications 1 à 4, caractérisé en ce que l'on hydrolyse l'amino ester de formule générale II:

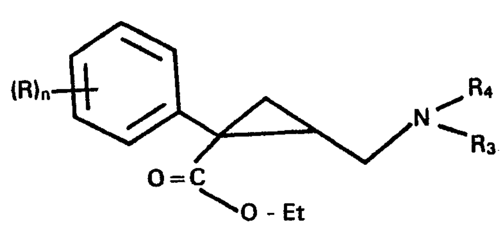

(II)

pour obtenir les aminoacides de formule générale III:

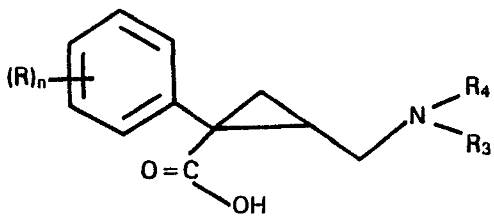

(III)

ces dérivés de formule générale III étant transformés en halogénure de formule générale IV par un halogénure de thionyle SO $X_2$:

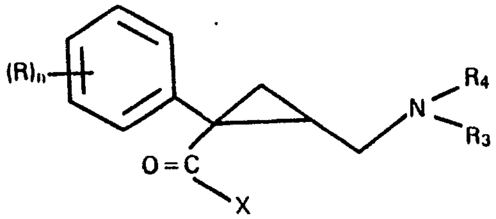

(IV)

les dérivés de formule générale IV étant ensuite traités par une amine de formule:

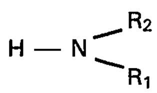

pour donner les composés de formule générale I;

X représentant un atome d'halogène et

R, $R_1$, $R_2$, $R_3$, $R_4$ et n ayant les significations données à la revendication 1.

6. A titre de médicaments nouveaux les composés selon l'une des revendications 1 à 4.

7. Les compositions pharmaceutiques caractérisées en ce qu'elles contiennent comme principe actif au moins un composé selon l'une des revendications 1 à 4.

**Revendications pour l'Etat contractant:** AT

1. Procédé de préparation de dérivés d'aryl-1 aminométhyl-2 cyclopropanes carboxamides (Z) répondant à la formule générale I:

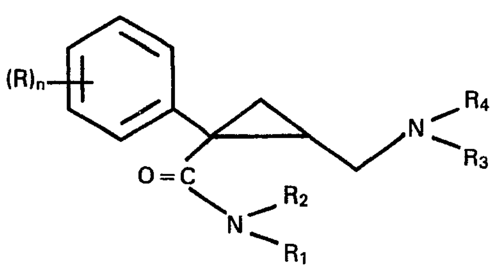

(I)

dans laquelle:

R représente un atome d'hydrogène ou d'halogène, un groupe alcoyle inférieur en $C_1$ à $C_4$, alcoxy inférieur en $C_1$ à $C_4$, hydroxy, nitro et amino;

n représente les valeurs 1 ou 2;

$R_1$ et $R_2$ représentent un atome d'hydrogène, un groupe alcoyle inférieur en $C_1$ à $C_4$, un groupe aryle ou alcoylaryle dont le fragment alcoyle contient 1 à 4 atomes de carbone, éventuellement substitués, de préférence en position para, par un atome d'halogène, de préférence par un atome de chlore;

$R_1$ et $R_2$ pouvant également former avec l'atome d'azote voisin un hétérocycle à 5 ou 6 chaînons;

$R_3$ et $R_4$ représentent un atome d'hydrogène ou un groupe alcoyle inférieur en $C_1$ à $C_4$;

$R_3$ et $R_4$ pouvant également former avec l'atome d'azote voisin un hétérocycle à 5 ou 6 chaînons, contenant éventuellement un hétéroatome supplémentaire choisi parmi l'azote et l'oxygène,

ainsi que leurs sels avec des acides minéraux ou organiques thérapeutiquement acceptables,

caractérisé en ce que l'on hydrolyse l'amino ester de formule générale II:

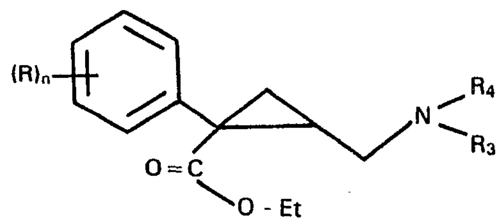

(II)

pour obtenir les aminoacides de formule générale III:

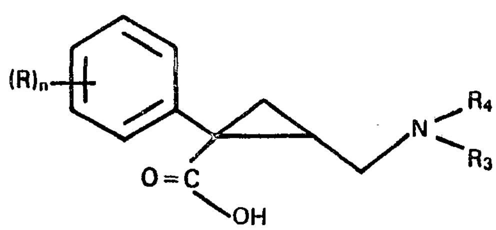

(III)

ces dérivés de formule générale III étant transformés en halogénure de formule générale IV par un halogénure de thionyle SO $X_2$:

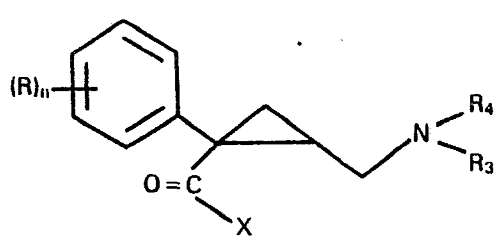

(IV)

les dérivés de formule générale IV étant ensuite traités par une amine de formule:

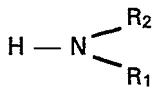

pour donner les composés de formule générale I;

X représentant un atome d'halogène et

R, $R_1$, $R_2$, $R_3$, $R_4$ et n ayant les significations données à la revendication 1.

2. Procédé selon la revendication 1, caractérisé en ce que l'on prépare le chlorhydrate de phényl-1 diéthylaminocarbonyl-1 aminométhyl-2 cyclopropane (Z).

3. Procédé selon la revendication 1, caractérisé en ce que l'on prépare le chlorhydrate de p-chlorophényl-1 aminocarbonyl-1 aminométhyl-2 cyclopropane (Z).

4. Procédé selon la revendication 1, caractérisé en ce que l'on prépare le chlorhydrate de p-méthoxy phényl-1 amino carbonyl-1 aminométhyl-2 cyclopropane (Z).

**Patentansprüche für die Vertragsstaaten:**
BE, CH, DE, GB, IT, LI, LU, NL, SE

1. Derivate von Aryl-1-aminomethyl-2-cyclopropan-carboxamiden (Z) der allgemeinen Formel (I),

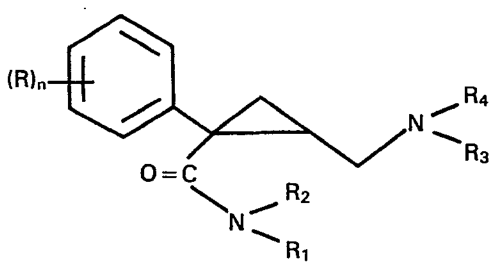
(I)

worin

R ein Wasserstoff- oder Halogenatom, eine niedere Alkylgruppe mit 1 bis 4 Kohlenstoffatomen, eine niedere Alkoxygruppe mit 1 bis 4 Kohlenstoffatomen, Hydroxy, Nitro und Amino bedeutet,

n die Zahlen 1 oder 2 bedeutet,

$R_1$ und $R_2$ ein Wasserstoffatom, eine niedere Alkylgruppe mit 1 bis 4 Kohlenstoffatomen, eine Aryl- oder Alkylarylgruppe bedeuten, deren Alkylrest 1 bis 4 gegebenenfalls mit einem Halogenatom, vorzugsweise einem Chloratom, vorzugsweise in para-Stellung substituierte Kohlenstoffatome aufweist,

wobei $R_1$ und $R_2$ mit dem benachbarten Stickstoffatom ebenso einen 5- oder 6-gliedrigen Heterocyclus bilden können,

$R_3$ und $R_4$ ein Wasserstoffatom oder eine niedere Alkylgruppe mit 1 bis 4 Kohlenstoffatomen bedeuten,

wobei $R_3$ und $R_4$ mit dem benachbarten Stickstoffatom ebenso einen 5- oder 6-gliedrigen Heterocyclus bilden können, welcher gegebenenfalls ein zusätzliches, aus Stickstoff und Sauerstoff ausgewähltes Heteroatom aufweist,

sowie deren therapeutisch annehmbare Salze mit Mineralsäuren oder organischen Säuren.

2. Hydrochlorid von Phenyl-1-diethylaminocarbonyl-1-aminomethyl-2-cyclopropan (Z) nach Anspruch 1.

3. Hydrochlorid von p-Chlorphenyl-1-aminocarbonyl-1-aminomethyl-2-cyclopropan (Z) nach Anspruch 1.

4. Hydrochlorid von p-Methoxyphenyl-1-aminocarbonyl-1-aminomethyl-2-cyclopropan (Z) nach Anspruch 1.

5. Verfahren zur Herstellung von Verbindungen der allgemeinen Formel (I) nach einem der Ansprüche 1 bis 4, dadurch gekennzeichnet, dass man den Aminosäureester der allgemeinen Formel (II)

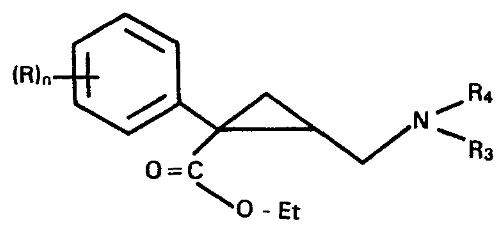
(II)

hydrolysiert, um die Aminosäuren der allgemeinen Formel (III)

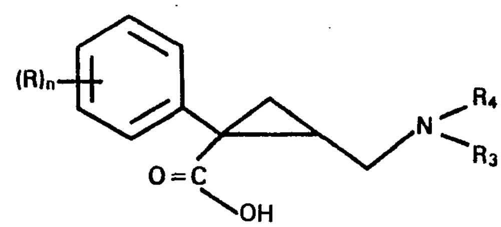
(III)

zu erhalten, worauf diese Derivate der allgemeinen Formel (III) mittels eines Thionylhalogenides $SOX_2$ zu Halogeniden der allgemeinen Formel (IV)

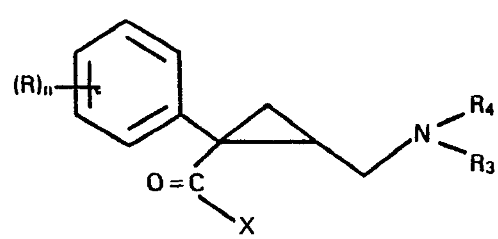
(IV)

umgesetzt werden und die Derivate der allgemeinen Formel (IV) daraufhin mit einem Amin der Formel

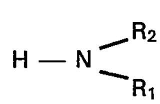

zu Verbindungen der allgemeinen Formel (I) umgesetzt werden, worin

X ein Halogenatom bedeutet und

R, $R_1$, $R_2$, $R_3$, $R_4$ und n die in Anspruch 1 angegebenen Bedeutungen haben.

6. Verbindungen nach einem der Ansprüche 1 bis 4 als neue Medikamente.

7. Pharmazeutische Zusammensetzungen, dadurch gekennzeichnet, dass sie als wirksames Prinzip wenigstens eine Verbindung nach einem der Ansprüche 1 bis 4 enthalten.

**Patentansprüche für den Vertragsstaat:** AT

1. Verfahren zur Herstellung von Derivaten von Aryl-1-aminomethyl-2-cyclopropan-carboxamiden (Z) der allgemeinen Formel (I),

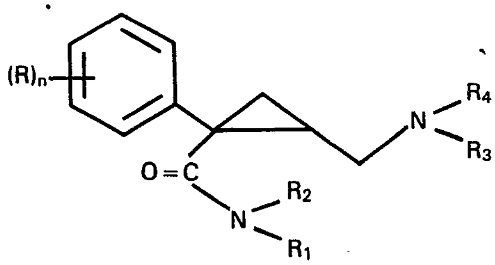
(I)

worin

R ein Wasserstoff- oder Halogenatom, eine niedere Alkylgruppe mit 1 bis 4 Kohlenstoffatomen, eine niedere Alkoxygruppe mit 1 bis 4 Kohlenstoffatomen, Hydroxy, Nitro und Amino bedeutet,

n die Zahlen 1 oder 2 bedeutet,

$R_1$ und $R_2$ ein Wasserstoffatom, eine niedere Alkylgruppe mit 1 bis 4 Kohlenstoffatomen, eine Aryl- oder Alkylarylgruppe bedeuten, deren Alkylrest 1 bis 4 gegebenenfalls mit einem Halogenatom, vorzugsweise einem Chloratom, vorzugsweise in para-Stellung substituierte Kohlenstoffatome aufweist,

wobei $R_1$ und $R_2$ mit dem benachbarten Stickstoffatom ebenso einen 5- oder 6-gliedrigen Heterocyclus bilden können,

$R_3$ und $R_4$ ein Wasserstoffatom oder eine niedere Alkylgruppe mit 1 bis 4 Kohlenstoffatomen bedeuten,

wobei $R_3$ und $R_4$ mit dem benachbarten Stickstoffatom ebenso einen 5- oder 6-gliedrigen Heterocyclus bilden können, welcher gegebenenfalls ein zusätzliches, aus Stickstoff und Sauerstoff ausgewähltes Heteroatom aufweist,

sowie deren therapeutisch annehmbaren Salzen mit Mineralsäuren oder organischen Säuren, dadurch gekennzeichnet, dass man den Aminosäureester der allgemeinen Formel (II)

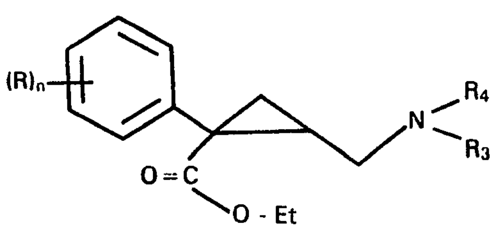
(II)

hydrolysiert, um die Aminosäuren der allgemeinen Formel (III)

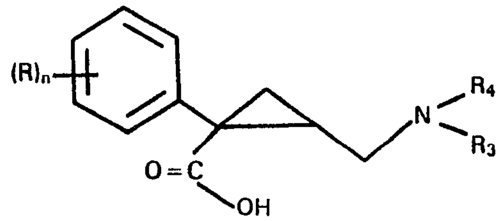
(III)

zu erhalten, worauf diese Derivate der allgemeinen Formel (III) mittels eines Thionylhalogenides $SOX_2$ zu Halogeniden der allgemeinen Formel (IV)

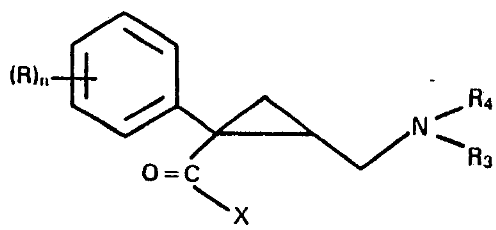
(IV)

umgesetzt werden und die Derivate der allgemeinen Formel (IV) daraufhin mit einem Amin der Formel

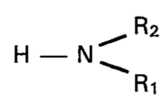

zu Verbindungen der allgemeinen Formel (I) umgesetzt werden, worin

X ein Halogenatom bedeutet und

R, $R_1$, $R_2$, $R_3$, $R_4$ und n die in Anspruch 1 angegebenen Bedeutungen haben.

2. Verfahren nach Anspruch 1, dadurch gekennzeichnet, dass man das Hydrochlorid von Phenyl-1-diethylaminocarbonyl-1-aminomethyl-2-cyclopropan (Z) herstellt.

3. Verfahren nach Anspruch 1, dadurch gekennzeichnet, dass man das Hydrochlorid von p-Chlorphenyl-1-aminocarbonyl-1-aminomethyl-2-cyclopropan (Z) herstellt.

4. Verfahren nach Anspruch 1, dadurch gekennzeichnet, dass man das Hydrochlorid von p-Methoxyphenyl-1-aminocarbonyl-1-aminomethyl-2-cyclopropan (Z) herstellt.

**Claims for the Contracting States:**
BE, CH, DE, GB, IT, LI, LU, NL, SE

1. Derivatives of 1-aryl-2-aminomethyl-cyclopropane-carboxamides (Z) corresponding to the general formula I:

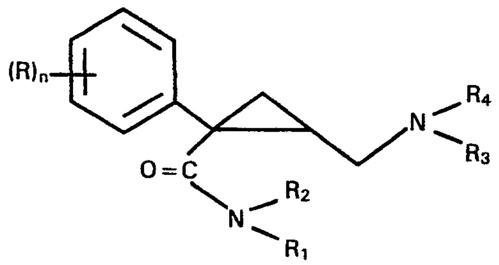
(I)

wherein,

R represents a hydrogen or halogen atom, a lower $C_1$-$C_4$ alkyl group, a lower $C_1$-$C_4$ alkoxy group, a hydroxy group, a nitro group and an amino group;

n represents the value 1 or 2;

$R_1$ and $R_2$ represent a hydrogen atom, a lower $C_1$-$C_4$ alkyl group, an aryl or alkyl-aryl group, the alkyl part of which has from 1 to 4 carbon atoms, optionally substituted, preferably in the para position, by a halogen atom, preferably by a chlorine atom;

$R_1$ and $R_2$ can also form, with the adjoining nitrogen atom, a heterocycle having 5 or 6 members;

$R_3$ and $R_4$ represent a hydrogen atom or a lower $C_1$-$C_4$ alkyl group;

$R_3$ and $R_4$ can also form, with the adjoining nitrogen atom, a heterocycle having 5 or 6 members, optionally containing an additional heteroatom selected from nitrogen and oxygen, as well as the salts thereof with therapeutically acceptable inorganic or organic acids.

2. 1-phenyl-1-diethylaminocarbonyl-2-aminomethyl-cyclopropane (z) hydrochloride according to claim 1.

3. 1-p-chlorophenyl-1-aminocarbonyl-2-aminomethyl-cyclopropane (z) hydrochloride according to claim 1.

4. 1-p-methoxy-phenyl-1-aminocarbonyl-2-aminomethyl-cyclopropane (z) hydrochloride according to claim 1.

5. A process for the preparation of compounds corresponding to the general formula I according to one of claims 1 to 4, characterised in that the amino ester corresponding to the general formula II:

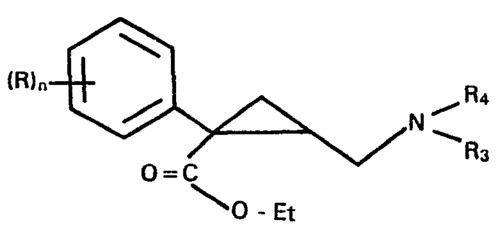
(II)

is hydrolyzed to produce amino acids corresponding to the general formula III:

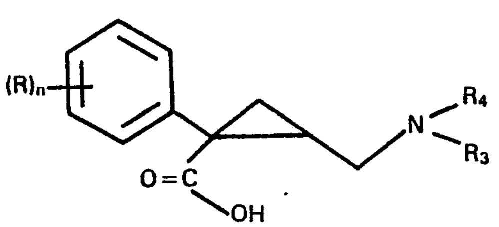
(III)

these derivatives corresponding to the general formula III being converted into halide corresponding to the general formula IV by a thionyl halide SO $X_2$:

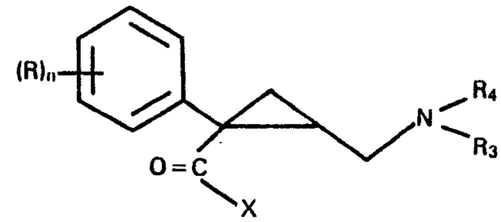

the derivatives corresponding to the general formula IV then being treated by an amine corresponding to the formula:

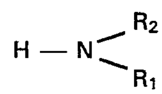

to produce the compounds corresponding to the general formulaI;

X representing a halogen atom and

R, $R_1$, $R_2$, $R_3$, $R_4$ and n being as defined in claim 1.

6. The compounds according to one of claims 1 to 4 as new medicaments.

7. Pharmaceutical compositions characterised in that they contain at least one compound according to one of claims 1 to 4 as active principle.

**Claims for the Contracting State:** AT

1. A process for the preparation of 1-aryl-2-aminomethyl-cyclopropane-carboxamides (z) corresponding to the general formula I:

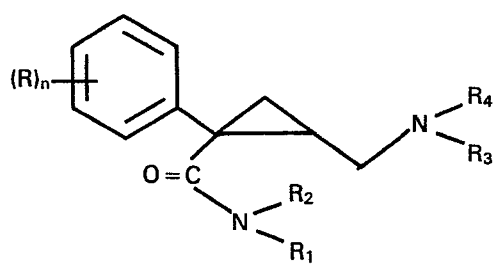
(I)

wherein,

R represents a hydrogen or halogen atom, a $C_1$-$C_4$ lower alkyl group, a $C_1$-$C_4$ lower alkoxy group, a hydroxy group, a nitro group and an amino group;

n represents the value 1 or 2;

$R_1$ and $R_2$ represent a hydrogen atom, a $C_1$-$C_4$ lower alkyl group, an aryl or alkyl-aryl group, the alkyl part of which has from 1 to 4 carbon atoms, optionally substituted, preferably in the para position, by a halogen atom, preferably by a chlorine atom;

$R_1$ and $R_2$ can also form, with the adjoining nitrogen atom, a heterocycle having 5 or 6 members;

$R_3$ and $R_4$ represent a hydrogen atom or a $C_1$-$C_4$ lower alkyl group;

$R_3$ and $R_4$ can also form, with the adjoining nitrogen atom, a heterocycle having 5 or 6 members, optionally containing an additional heteroatom selected from nitrogen and oxygen, as well as the salts thereof with therapeutically acceptable inorganic or organic acids, characterised in that the amino ester corresponding to the general formula II is hydrolyzed:

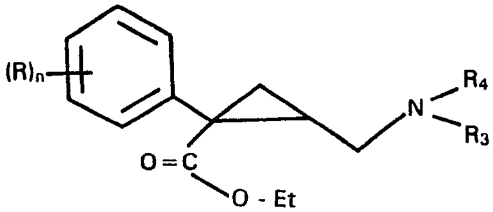 (II)

to produce amino acids corresponding to the general formula III:

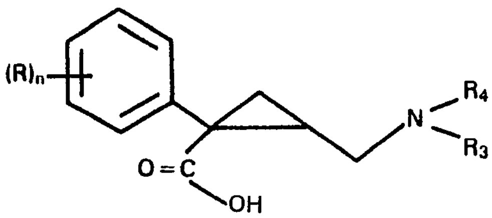 (III)

these derivatives corresponding to the general formula III being converted into halide corresponding to the general formula IV by a thionyl halide SO $X_2$:

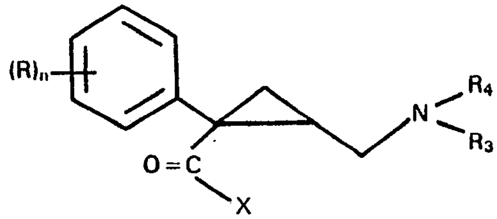 (IV)

the derivatives corresponding to the general formula IV then being treated by an amine corresponding to the formula:

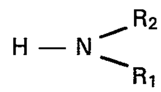

to produce the compounds corresponding to the general formulaI;

X representing a halogen atom and

R, $R_1$, $R_2$, $R_3$, $R_4$ and n being as defined in claim 1.

2. A process according to claim 1, characterised in that 1-phenyl-1-diethylaminocarbonyl-2-aminomethyl-cyclopropane (z) hydrochloride is prepared.

3. A process according to claim 1, characterised in that 1-p-chlorophenyl-1-aminocarbonyl-2-aminomethyl-cyclopropane (z) hydrochloride is prepared.

4. A process according to claim 1, characterised in that 1-p-methoxy-phenyl-1-aminocarbonyl-2--aminomethyl-cyclopropane (z) hydrochloride is prepared.